# FASCICULE DE BREVET EUROPEEN

(11) **EP 0 676 195 B1**
(45) Date de publication et mention de la délivrance du brevet: **06.05.1999**
(21) Numéro de dépôt: 95400110.3
(22) Date de dépôt: 19.01.1995
(51) Int. Cl.: A61K 7/48

(54) **Composition cosmétique pour masque de nettoyage de la peau contenant des particules de polyamide sphéroidales calibrées**
Kosmetische Zusammensetzung für Masken zur Reinigung der Haut, die sphäroidale Polyamid-Partikel vorgegebener Grösse enthält
Cosmetic composition for a skin cleansing mask containing graded spheroidal polyamide particles

(30) Priorité: 24.03.1994 FR 9403452
(43) Date de publication de la demande: 11.10.1995
(73) Titulaire: L'OREAL, 75008 Paris (FR)
(72) Inventeur: Bui-Bertrand, Lien, F-91600 Savigny sur Orge (FR); Louvet-Plaisant, Nathalie, F-94550 Chevilly Larue (FR)
(74) Mandataire: Dodin, Catherine

(56) Documents cités:
- EP-A- 0 282 823
- JP-A-61 249 919
- US-A- 4 309 411
- CHEMICAL ABSTRACTS, vol. 92, no. 18, 5 Mai 1980, Columbus, Ohio, US; abstract no. 152880, 'PREPARATION OF VINYL FACE MASKS CONTAINING TITANIUM DIOXIDE MODIFIED NYLON 12 POWDER' & COSMET. TOILETRIES, vol.94, no.12, 1979 pages 33 - 37 TOIDA ET AL.
- DATABASE WPI Week 7950, Derwent Publications Ltd., London, GB; AN 79-90055B & JP-A-54 140 714 (LION DENTIFRICE)
- DATABASE WPI Week 8002, Derwent Publications Ltd., London, GB; AN 80-02595C & JP-A-54 150 459 (LION DENTIFRICE)
- S.T.N., Serveur de bases de données, KARLSRUHE, DE, Fichier Chemical Abstracts, vol 99, n 218416 & JP-A-58069248 (LION CORP.) * résumé *

## Description

La présente invention se rapporte à une composition cosmétique à usage de masque de nettoyage comprenant, entre autre, comme agent nettoyant essentiel, des particules de polyamide sphéroïdales calibrées, dispersées dans un gel aqueux.

Cette composition cosmétique constitue un produit de nettoyage de la peau sous forme de masque, destiné aux soins du visage, d'une partie du visage ou du cou, afin notamment de nettoyer la peau en profondeur, par exemple par élimination des cellules mortes de la couche cornée de surface ou par élimination des corps gras présents en excès à la surface de la peau (le sébum par exemple), de raffermir la peau, de l'adoucir et/ou de lui faire subir un traitement particulier.

L'invention se rapporte aussi à l'utilisation de particules sphéroïdales dans une composition pour masque, et à l'utilisation de cette composition dans un traitement cosmétique.

Les produits du type masque de beauté sont bien connus dans le domaine de la cosmétique. Ils se présentent notamment sous forme de gel, d'émulsion ou de pâte. Il est décrit dans la littérature, par exemple dans "Cosmetic and Toiletry Formulations", Seconde édition, Ernest W. Flick 1992, différentes formules de ce type de masque. Ces masques de beauté peuvent être des masques hydratants sous forme de gel, qui n'ont pas vocation de nettoyage, mais qui procurent à la peau un certain confort.

On connaît également des masques de nettoyage généralement sous forme d'émulsion, ayant le désagrément de procurer à la peau une sensation de gras, et qui contiennent en général une charge de type argileux.

Tous ces types de masques utilisent généralement une charge en une proportion qui ne dépasse pas 10 % en poids par rapport au poids total de la composition. Ce sont ces charges qui par leur pouvoir absorbant fixent les composés gras de la surface de l'épiderme (sébum), entraînant un nettoyage en profondeur de la peau.

Enfin, il existe des masques de "peeling" qui sont, soit des empâtages de kaolinite et/ou de montmorillonite, soit des compositions filmifiantes à base d'alcool polyvinylique. Ces masques de "peeling", après application sur le visage, sèchent pour donner un film que l'on retire par lavage, nettoyage ou arrachage.

Pendant le temps de séchage, la couche cornée (ou *stratum corneum*) s'humidifie et s'assouplit ; par ailleurs, la peau se contracte légèrement. Ces masques présentent la propriété d'absorber les corps gras lorsqu'ils sont au contact de la peau, et lorsqu'ils sont retirés assurent un nettoyage en profondeur de la peau en même temps qu'un "peeling", entraînant notamment les cellules mortes des couches cornées de surface.

Ces compositions de "peeling" ont le désagrément de présenter une texture épaisse sous forme de pâte, difficile à appliquer et qui sèche durant la pause. Ce séchage induit sur la peau de la patiente un effet de tiraillement. Cet effet astringent est ressenti comme un inconfort.

Par ailleurs, on connaît des masques de nettoyage à forte teneur en kaolin (23%). Ces masques se présentent sous la forme d'un empâtage que l'on applique sur le visage. Pendant le temps d'application, supérieur à une dizaine de minutes, ces compositions sèchent et provoquent elles aussi un tiraillement de la peau, engendrant également un grand inconfort.

Une fois sec, ces masques présentent l'inconvénient d'être difficiles à éliminer.

Afin de supprimer les inconvénients ci-dessus, la demanderesse a cherché à réaliser des compositions cosmétiques présentant des qualités de nettoyage de la peau au moins égales aux masques connus dans le domaine cosmétique, sans qu'elles ne présentent les effets indésirables des empâtages ou des émulsions classiques.

C'est ainsi que de manière surprenante et inattendue, la demanderesse a constaté qu'il était possible d'incorporer dans des gels aqueux une forte proportion d'une charge constituée de particules de polyamide sphéroïdales calibrées, afin d'obtenir des compositions cosmétiques ayant l'aspect d'une crème, tout en conservant les propriétés intrinsèques des gels, à savoir l'apport d'une agréable sensation de fraîcheur à l'application sans effet gras, ces compositions pouvant être utilisées comme masque de nettoyage de la peau.

Après application sur le visage pendant un temps suffisant, à savoir entre 5 et 10 minutes, ces compositions présentent le grand avantage de pouvoir être retirées par simple lavage à l'eau et rinçage.

Par ailleurs, tout en présentant des propriétés de masque de nettoyage, ces compositions cosmétiques, après élimination, procurent un éclaircissement du teint. Enfin, on peut constater après application, un effet optique d'atténuation de la profondeur des rides.

Les particules de polyamide sphéroïdales calibrées utilisées dans la composition de l'invention sont connues pour leur utilisation dans le domaine cosmétique dans des fonds de teint ou crèmes. Cependant, les concentrations d'utilisation préconisées dans ces fonds de teint ou crèmes, n'excèdent jamais 10 % du poids total de la composition.

Par ailleurs, le brevet japonais n° 61-249919 de la Société SHISEIDO enseigne l'incorporation dans des gels de poudres organiques comme les poudres de Nylon, à de fortes teneurs, jusqu'à 40 % en poids. Cependant, les gels utilisés sont des gels à très haute teneur en agent gélifiant, de 5 % à 40 % du poids total du gel.

On obtient ainsi un produit dont la texture s'apparente à une pâte épaisse que l'on malaxe à la main avant utilisation et qui est très éloignée des produits d'aspect crémeux obtenus selon l'invention. Ce produit est en fait un cataplasme.

D'ailleurs, la demanderesse a pu constater qu'il était impossible d'obtenir une composition cosmétique ayant les propriétés de celles de l'invention (aspect crème, efficacité de nettoyage, facilité d'emploi et d'élimination, ne tire pas la peau) en utilisant, à la place des particules de polyamide et en proportion supérieure à 10 % en poids total de la composition, une charge du type kaolin.

De plus, la masse volumique des particules de polyamide étant nettement supérieure à celle des additifs classiques des compositions cosmétiques pour masques de nettoyage, il n'est pas du tout évident, pour l'homme de l'art, d'introduire dans les compositions traditionnelles sous forme de gel, servant de base aux masques, des quantités de particules de polyamide supérieures à 12 %, en conservant à ces compositions des qualités de gels.

L'invention a donc pour objet une composition cosmétique gélifiée à usage de masque de nettoyage de la peau, caractérisée par le fait qu'elle comprend, entre autre, un gel aqueux comprenant au moins un agent gélifiant dans un véhicule aqueux, ledit agent gélifiant étant présent en une quantité allant de 0,3 à 1 % du poids total de la composition, et, comme agent de nettoyage principal, des particules de polyamide sphéroïdales calibrées dispersées dans ce gel aqueux, lesdites particules étant en une proportion au moins égale à 12 % en poids par rapport au poids total de la composition, ayant une densité allant de 1,0 g/cm³ à 1,84 g/cm³ et une granulométrie de 5-50 µm.

Cette composition, bien qu'ayant l'aspect d'une crème, présente des propriétés très différentes de celles d'une crème de soin. En particulier, les effets d'un masque de nettoyage apparaissent après son élimination et non pendant son application sur le visage, à l'inverse d'une crème.

De plus, cette composition pour masque ne sèche pas pendant la pose et n'entraîne donc pas de tiraillements de la peau comme les compositions de l'art antérieur à fort taux de charge.

En règle générale, les polyamides utilisées sont répertoriées sous le nom CTFA de "*Nylon 12*" ou "*Nylon 6*".

Les particules de polyamide utilisées dans l'invention peuvent être celles vendues sous la dénomination "ORGASOL" par la Société ATOCHEM. Le procédé d'obtention de ces particules est par exemple celui décrit dans le document FR 2 619 385 ou dans le document EP 303 530. Ces particules de polyamide sont par ailleurs connues selon leurs différentes propriétés physico-chimiques sous le nom de "*polyamide 12*" ou "*polyamide 6*".

Selon l'invention, les particules sont utilisées de préférence à raison de 12 % à 35 % du poids total de la composition et mieux, à raison de 15 % à 20 %.

Malgré le taux élevé de charges, la composition pour masque de l'invention est stable au moins 2 mois à 45 °C, contrairement à ce qui se passe avec les autres charges minérales ou organiques.

Les particules utilisées dans l'invention peuvent également être celles vendues sous la dénomination SP500 par la Société KOBO.

Dans les compositions selon l'invention, les particules ont une densité allant de 1,0 g/cm³ à 1,84 g/cm³ et en particulier, une densité allant de 1,02 g/cm³ à 1,4 g/cm³.

Les particules de l'invention sont sphériques et pleines ; elles ont une granulométrie allant de 5 µm à 50 µm et plus particulièrement allant de 10 µm à 30 µm.

Pour réaliser le gel qui est à la base de la composition, on utilise au moins un agent gélifiant dans un véhicule liquide aqueux. Bien entendu, l'agent gélifiant est présent en quantité suffisante pour conférer à la composition la viscosité souhaitée. En principe, cette dernière doit être supérieure à 2 Pa.s. Ainsi, on introduit dans la composition l'agent gélifiant en une proportion allant de 0,3 % à 1 % en poids par rapport au poids total de la composition et de préférence de 0,5 % à 0,8 %.

Les agents gélifiants sont ceux classiquement utilisés dans le domaine cosmétique, et en particulier ceux choisis parmi les polymères hydrosolubles et ceux donnant, dans l'eau, des solutions colloïdales.

Ce sont notamment les polymères ou copolymères d'acides organiques carboxyliques insaturés ou d'esters insaturés, les dérivés polysaccharidiques, les gommes, les silicates colloïdaux, les polyéthylèneglycols (PEG) et leurs dérivés, les polyvinylpyrrolidones et leurs dérivés, et les gels de silice hydrophiles.

Les agents gélifiants sont par exemple les polymères ou copolymères acryliques et/ou méthacryliques, les polymères carboxyvinyliques, les acrylates ou méthacrylates de polyglycéryle, les dérivés de cellulose, d'amidon ou de chitine, les alginates, l'acide hyaluronique et ses sels, les chondroïtine sulfates, les gommes de xanthane, de gellane, de rhamsan, de karoya, ou de guar, la farine de caroube, et les silicates d'aluminium et de magnésium colloïdaux de type montmorillonite.

On peut citer notamment comme agents gélifiants particuliers : les polymères carboxyvinyliques vendus sous la dénomination CARBOPOL par la Société GOODRICH ou le SYNTHALEN K par la Société SIGMA, les copolymères acide acrylique/acrylate d'éthyle, les copolymères acide acrylique/méthacrylate de stéaryle, le polyglycérylméthacrylate vendu sous la dénomination LUBRAJEL par la Société GUARDIAN, le polyglycérylacrylate vendu sous la dénomination HISPAGEL par la Société HISPANO CHIMICA, la carboxyméthylcellulose, I'hydroxyméthylcellulose, l'hydroxypropylcellulose, la cellulose microcristalline, l'hydroxypropylguar, les hectorites ou bentonites colloïdales vendues sous la dénomination VEEGUM par la Société VAN DER BILT.

Les compositions de l'invention peuvent aussi contenir les ingrédients additionnels hydrosolubles ou solubilisés dans l'eau utilisés habituellement dans les compositions cosmétiques, comme des polyols, des agents conservateurs, des agents hydratants, des parfums, des peptisants ou solubilisants des parfums comme le CREMOPHOR R460 de la Société BASF, ou l'huile de castor, des agents de texture tels que des agents pulvérulents, des colorants. L'avantage des polyols est d'améliorer, encore, I'élimination du masque par simple rinçage.

Parmi les ingrédients hydrosolubles ou solubilisés dans l'eau utilisables, on peut citer en particulier, le propylèneglycol, le dipropylèneglycol, le butylène-1,3 glycol, la glycérine, la polyglycérine, le sorbitol, le glucose, le saccharose, le gluconate de magnésium, les oligo-éléments, les acides siliconés hydrosolubles.

Ces ingrédients sont utilisés dans des proportions classiques dans le domaine cosmétique. En particulier, les polyols représentent de 0,1 % à 50 % du poids total de la composition et mieux de 5 % à 15 %, alors que les autres ingrédients additionnels représentent, chacun, jusqu'à 2 % du poids total de la composition.

Certaines compositions de l'invention peuvent également contenir des charges pulvérulentes, en particulier du talc, du kaolin, des argiles du type montmorillonite, l'hectorite ou la bentonite, d'autres charges telles que les silices ou les poudres de silicone (TOSPEARL de la Société TOSHIBA) ou la poudre de polyméthacrylate de méthyle (MICROPEARL de la Société MATSUMOTO) pour obtenir des effets d'optique, des microsphères creuses expansées (EXPANCEL de la Société KEMANORD), de la poudre d'amidon de maïs modifiée ou de la poudre de soie. Ces charges représentent moins de 5 % du poids total de la composition.

On peut également envisager d'incorporer aux compositions cosmétiques selon l'invention une dispersion de corps gras sans pour cela atteindre la forme d'une émulsion. Ces corps gras peuvent être par exemple des esters d'acide gras, du beurre de karité, des esters d'acide gras polyoxyéthylénés, des huiles végétales et minérales.

Pour préparer les compositions selon l'invention, on dissout dans l'eau à 90 °C les éventuels additifs de la composition (conservateurs, parex.) et/ou les ingrédients solubles tels les polyols que l'on désire ajouter.

On disperse ensuite l'agent gélifiant dans la solution aqueuse sous forte agitation. Lorsque l'agent gélifiant est totalement dissout, on ramène la température aux alentours de 50 °C et on incorpore les particules de polyamide sous agitation forte afin d'homogénéiser l'ensemble. Puis on refroidit la composition avant de neutraliser son pH (voisin de celui de la peau). On colore et parfume la composition à température ambiante.

L'invention a également pour objet l'utilisation de particules de polyamide sphéroïdales calibrées telles que définies précédemment comme additif dans une composition cosmétique ayant l'aspect d'un gel utilisée comme masque de nettoyage de la peau, notamment du visage, pour lui conférer les qualités d'une crème.

En outre, l'invention a aussi pour objet l'utilisation des compositions définies précédemment pour le nettoyage de la peau du visage.

Enfin, l'invention a également pour objet un procédé de traitement cosmétique caractérisé par le fait que l'on applique sur la peau une composition cosmétique telle que définie précédemment.

On donne maintenant à titre d'exemples, mais de manière non limitative, des formules de compositions conformes à l'invention. Les quantités de chaque constituant sont données en pourcentage pondéral.

### EXEMPLE 1

- Orgasol 2002 D 22 %
- Synthalen K 0,55 %
- Soude 0,22 %
- Butylène-1,3 glycol 25 %
- Conservateur 0,3 %
- Colorant 0,007 %
- Parfum 0,2 %
- Peptisant 0,2 %
- Eau qsp 100 %

On obtient un gel crème épais, lisse et brillant, facile à étaler, qui ne sèche pas pendant la pose (5 à 7min.), et ne procure donc pas de sensation d'inconfort (tiraillement). Il s'élimine facilement à l'eau et laisse une peau douce, nettoyée et mate.

### EXEMPLE 2

- Orgasol/2002 D 17 %
- Carbopol 980 0,5 %
- Triéthanolamine 0,5 %
- Propylène glycol 20 %
- Conservateurs 0,3 %
- Parfum 0,2 %
- Peptisant 0,2 %
- Eau qsp 100 %

On obtient un gel crème épais, lisse et brillant, de mêmes caractéristiques que le précédent.

### EXEMPLE 3

- SP 500 22 %
- Synthalen K 0,55 %
- Soude 0,22 %
- Butylène-1,3 glycol 25 %
- Conservateurs 0,3 %
- Parfum 0,2 %
- Peptisant 0,2 %
- Eau qsp 100 %

On obtient une crème souple, lisse et brillante. L'utilisation et l'efficacité sont identiques aux deux précédents.

### EXEMPLE 4

- Orgasol 2002 D 22 %
- Synthalen K 0,55 %
- Soude 0,22 %
- Conservateur 0,3 %
- Parfum 0,2 %
- Peptisant 0,2 %
- Colorant 0,007 %
- Eau qsp 100 %

On obtient un gel crème épais, lisse, moins brillant que l'exemple 1 ; ceci est dû à l'absence des glycols.

A titre d'essais comparatifs, la demanderesse a cherché à réaliser des compositions selon l'invention, en remplaçant dans l'exemple 1 la poudre de polyamide par du kaolin à des proportions de 11 % et 22 % du poids de la composition. Le produit obtenu s'est révélé grumeleux et caoutchouteux, sans ressemblance avec les gels d'aspect crémeux obtenus selon l'invention. Le produit ainsi obtenu n'adhère pas à la peau et n'est pas du tout utilisable dans le domaine cosmétique. En fait, l'introduction de grandes quantités de charge désorganise la structure du gel.

## Revendications

1. Composition cosmétique gélifiée à usage de masque de nettoyage de la peau, caractérisée par le fait qu'elle comprend, entre autre, un gel aqueux comprenant au moins un agent gélifiant dans un véhicule aqueux, ledit agent gélifiant étant présent en une quantité allant de 0,3 à 1 % du poids total de la composition, et, comme agent de nettoyage principal, des particules de polyamide sphéroïdales calibrées dispersées dans ce gel aqueux, lesdites particules étant en une proportion au moins égale à 12 % en poids par rapport au poids total de la composition, ayant une densité allant de 1,0 g/cm³ à 1,84 g/cm³ et une granulométrie de 5-50 µm.

2. Composition cosmétique selon la revendication 1, caractérisée par le fait que les particules sont en une proportion allant de 12 % à 35 % en poids par rapport au poids total de la composition.

3. Composition cosmétique selon la revendication 1 ou 2, caractérisée par le fait que les particules ont une densité de 1,02-1,4 g/cm³.

4. Composition cosmétique selon l'une quelconque des revendications précédentes, caractérisée par le fait que les particules ont une granulométrie de 10 µm à 30 µm.

5. Composition cosmétique selon l'une quelconque des revendications précédentes, caractérisée par le fait que l'agent gélifiant est choisi parmi les polymères hydrosolubles et les polymères donnant dans de l'eau des solutions colloïdales.

6. Composition cosmétique selon la revendication 5, caractérisée par le fait que l'agent gélifiant est choisi parmi les polymères et copolymères d'acide organique carboxylique insaturé ou d'ester insaturé, les dérivés polysaccharidiques, les gommes, les silicates colloïdaux, les polyéthylènes glycol (PEG) et leurs dérivés, les polyvinylpyrrolidones et leurs dérivés, les gels de silice hydrophiles.

7. Composition cosmétique selon l'une quelconque des revendications 5 ou 6, caractérisée par le fait que l'agent gélifiant est choisi parmi les polymères et copolymères acryliques et/ou méthacryliques, les polymères carboxyvinyliques, les acrylates ou méthacrylates de polyglycéryle, les dérivés de cellulose, d'amidon et de chitine, les alginates, l'acide hyaluronique et ses sels, les chondroïtine sulfates, les gommes de xanthane, de gellane, de rhamsan, de karoya ou de guar, la farine de caroube et les silicates d'aluminium et de magnésium colloïdaux.

8. Composition cosmétique selon l'une quelconque des revendications 1 à 7, caractérisée par le fait que l'agent gélifiant est présent dans une proportion allant de 0,5 % à 0,8 % du poids total de la composition.

9. Composition cosmétique selon l'une quelconque des revendications précédentes, caractérisée par le fait qu'elle contient, en outre, des additifs hydrosolubles.

10. Composition cosmétique selon la revendication 9, caractérisée par le fait que les additifs hydrosolubles sont choisis parmi la glycérine, le propylène glycol, le dipropylène glycol, le butylène-1,3 glycol, la polyglycérine, le sorbitol, le glucose, le saccharose, le gluconate de magnésium, les oligo-éléments, les acides siliconés hydrosolubles.

11. Composition cosmétique selon l'une quelconque des revendications précédentes, caractérisée par le fait qu'elle contient, en outre, une charge différente desdites particules, représentant moins de 5 % du poids total de la composition.

12. Composition cosmétique selon la revendication 11, caractérisée par le fait que la charge est choisie parmi le talc, le kaolin, les microsphères creuses expansées, la poudre d'amidon de maïs modifiée, la poudre de soie, les argiles, les silices, les poudres de silicones, la poudre de polyméthacrylate de méthyle.

13. Utilisation de particules de polyamides sphéroïdales calibrées ayant une densité allant de 1,0 g/cm³ à 1,84 g/cm³ et une granulométrie de 5-50 µm comme additif dans une composition cosmétique d'aspect gélifié à usage de masque de nettoyage de la peau du visage pour lui conférer les qualités d'une crème.

14. Utilisation selon la revendication 13, caractérisée en ce que les particules représentent au moins 12 % du poids du poids total de la composition.

15. Utilisation selon la revendication 14, caractérisée par le fait que les particules sont en une proportion allant de 12 % à 35 % en poids par rapport au poids total de la composition.

16. Utilisation selon l'une quelconque des revendications 13 à 15, caractérisée par le fait que les particules ont une densité allant de 1,02 g/cm³ à 1,4 g/cm³.

17. Utilisation selon l'une quelconque des revendications 13 à 16, caractérisée par le fait que les particules ont une granulométrie allant de 10 µm à 30 µm.

18. Utilisation cosmétique de la composition selon l'une quelconque des revendications 1 à 12 pour nettoyer la peau du visage.

19. Procédé de traitement cosmétique, caractérisé par le fait que l'on applique sur la peau du visage une composition cosmétique telle que définie dans l'une quelconque des revendications 1 à 12.

## Claims

1. Gelled cosmetic composition for use as a skin cleansing mask, characterized in that it comprises, inter alia, an aqueous gel comprising at least one gelling agent in an aqueous vehicle, the said gelling agent being present in an amount ranging from 0.3 to 1 % of the total weight of the composition, and, as principal cleansing agent, spheroidal polyamide particles of specific size which are dispersed in this agueous gel, the said particles being in a proportion greater than or equal to 12 % by weight relative to the total weight of the composition and having a density ranging from 1.0 g/cm³ to 1.84 g/cm³ and a particle size of 5-50 µm.

2. Cosmetic composition according to Claim 1, characterized in that the particles are in a proportion which ranges from 12 % to 35 % by weight relative to the total weight of the composition.

3. Cosmetic composition according to Claim 1 or 2, characterized in that the particles have a density of 1.02 - 1.4 g/cm³.

4. Cosmetic composition according to any one of the preceding claims, characterized in that the particles have a size of from 10 µm to 30 µm.

5. Cosmetic composition according to any one of the preceding claims, characterized in that the gelling agent is chosen from water-soluble polymers and polymers which give colloidal solutions in water.

6. Cosmetic composition according to Claim 5, characterized in that the gelling agent is chosen from polymers and copolymers of unsaturated carboxylic organic acid or of unsaturated ester, polysaccharide derivatives, gums, colloidal silicates, polyethylene glycols (PEGs) and derivatives thereof, polyvinylpyrrolidones and derivatives thereof, and hydrophilic silica gels.

7. Cosmetic composition according to either of Claims 5 and 6, characterized in that the gelling agent is chosen from acrylic and/or methacrylic polymers and copolymers, carboxyvinyl polymers, polyglyceryl acrylates or methacrylates, cellulose derivatives, starch derivatives and chitin derivatives, alginates, hyaluronic acid and its salts, chondroitin sulphates, xanthan gum, gellan gum, rhamsan gum, karoya gum or guar gum, carob flour, and colloidal silicates of aluminium and magnesium.

8. Cosmetic composition according to any one of Claims 1 to 7, characterized in that the gelling agent is present in a proportion which ranges from 0.5 % to 0.8 % of the total weight of the composition.

9. Cosmetic composition according to any one of the preceding claims, characterized in that it additionally contains water-soluble additives.

10. Cosmetic composition according to Claim 9, characterized in that the water-soluble additives are chosen from glycerol, propylene glycol, dipropylene glycol, 1,3-butylene glycol, polyglycerol, sorbitol, glucose, sucrose, magnesium gluconate, trace elements, and water-soluble silicone-containing acids.

11. Cosmetic composition according to any one of the preceding claims, characterized in that it additionally contains a filler other than the said particles and representing less than 5 % of the total weight of the composition.

12. Cosmetic composition according to Claim 11, characterized in that the filler is chosen from talc, kaolin, expanded hollow microspheres, modified cornstarch powder, silk powder, clays, silicas, silicone powders, and polymethyl methacrylate powders.

13. Use of spheroidal polyamide particles of a specific size having a density which ranges from 1.0 g/cm³ to 1.84 g/cm³ and a particle size of 5-50 µm as additive in a cosmetic composition of gelled appearance for use as a facial skin-cleansing mask, in order to impart to it the qualities of a cream.

14. Use according to Claim 13, characterized in that the particles make up at least 12 % of the total weight weight of the composition.

15. Use according to Claim 14, characterized in that the particles are in a proportion which ranges from 12 % to 35 % by weight relative to the total weight of the composition.

16. Use according to any one of Claims 13 to 15, characterized in that the particles have a density which ranges from 1.02 g/cm³ to 1.4 g/cm³.

17. Use according to any one of Claims 13 to 16, characterized in that the particles have a size which ranges from 10 µm to 30 µm.

18. Cosmetic use of the composition according to any one of Claims 1 to 12 for cleansing facial skin.

19. Method of cosmetic treatment, characterized in that a cosmetic composition as defined in any one of Claims 1 to 12 is applied to the facial skin.

## Patentansprüche

1. Kosmetische Zusammensetzung in Gelform zur Verwendung für Masken für die Hautreinigung, dadurch gekennzeichnet, daß sie unter anderem ein wäßriges Gel, das mindestens einen Gelbildner in einem wäßrigen Vehikel enthält, wobei der Gelbildner in einer Menge von 0,3 bis 1 % des Gesamtgewichts der Zusammensetzung enthalten ist, und als wesentliches Reinigungsmittel sphäroidale Polyamid-Partikel vorgegebener Größe enthält, die in diesem wäßrigen Gel dispergiert sind, wobei diese Partikel in einem Anteil von mindestens 12 Gew.-%, bezogen auf das Gesamtgewicht der Zusammensetzung, enthalten sind und eine Dichte von 1,0 g/cm³ bis 1,84 g/cm³ und eine Partikelgröße von 5-50 µm aufweisen.

2. Kosmetische Zusammensetzung nach Anspruch 1, dadurch gekennzeichnet, daß die Partikel in einem Anteil von 12 bis 35 Gew.-%, bezogen auf das Gesamtgewicht der Zusammensetzung, enthalten sind.

3. Kosmetische Zusammensetzung nach Anspruch 1 oder 2, dadurch gekennzeichet, daß die Partikel eine Dichte von 1,02 bis 1,4 g/cm³ aufweisen.

4. Kosmetische Zusammensetzung nach einem der vorhergehenden Ansprüche, dadurch gekennzeichnet, daß die Partikel eine Partikelgröße von 10 bis 30 µm aufweisen.

5. Kosmetische Zusammensetzung nach einem der vorhergehenden Ansprüche, dadurch gekennzeichnet, daß der Gelbildner unter wasserlöslichen Polymeren und Polymeren, die in Wasser kolloidale Lösungen ergeben, ausgewählt ist.

6. Kosmetische Zusammensetzung nach Anspruch 5, dadurch gekennzeichnet, daß der Gelbildner unter Polymeren und Copolymeren ungesättigter organischer Carbonsäuren oder ungesättigter Ester, Polysaccharid-Derivaten, Gummen, kolloidalen Silicaten, Polyethylenglykolen (PEG) und ihren Derivaten, Polyvinylpyrrolidonen und ihren Derivaten, hydrophilen Kieselsäuregelen ausgewählt ist.

7. Kosmetische Zusammensetzung nach einem der Ansprüch 5 und 6, dadurch gekennzeichnet, daß der Gelbildner unter den Acryl- und/oder Methacryl-Polymeren, den Acryl- und/oder Methacryl-Copolymeren, den Carboxyvinylpolymeren, den Polyglycerinacrylaten und -methacrylaten, den Cellulose-Derivaten, Stärke-Derivaten und Chitin-Derivaten, den Alginaten, der Hyaluronsäure und ihren Salzen, den Chondroitinsulfaten, Xanthangummi, Gellangummi, Rhamsangummi, Karoyagummi und Guargummi, Johannisbrotbaumkernmehl und kolloidalem Aluminiumsilicat und kolloidalem Magnesiumsilicat ausgewählt ist.

8. Kosmetische Zusammensetzung nach einem der Ansprüche 1 bis 7, dadurch gekennzeichnet, daß der Gelbildner in einem Anteil von 0,5 bis 0,8 % des Gesamtgewichts der Zusammensetzung enthalten ist.

9. Kosmetische Zusammensetzung nach einem der vorhergehenden Ansprüche, dadurch gekennzeichnet, daß sie außerdem wasserlösliche Zusätze enthält.

10. Kosmetische Zusammensetzung nach Anspruch 9, dadurch gekennzeichnet, daß die wasserlösliche Zusätze unter Glycerin, Propylenglykol, Dipropylenglykol, 1,3-Butylenglykol, Polyglycerin, Sorbit, Glucose, Saccharose, Magnesiumgluconat, den Spurenelementen, den wasserlöslichen silicium- bzw. silicongruppenhaltigen Säuren ausgewählt sind.

11. Kosmetische Zusammensetzung nach einem der vorhergehenden Ansprüche, dadurch gekennzeichnet, daß sie außerdem einen von den obigen Partikeln verschiedenen Füllstoff enthält, der weniger als 5 % des Gesamtgewichts der Zusammensetzung ausmacht.

12. Kosmetische Zusammensetzung nach Anspruch 11, dadurch gekennzeichnet, daß der Füllstoff unter Talk, Kaolin, expandierten hohlen Mikrokügelchen, modifiziertem Maisstärkepulver, Seidenpulvern, Tonen, Kieselsäuren, Siliconpulvern, Polymethylmethacrylatpulver ausgewählt ist.

13. Verwendung sphäroidaler Polyamid-Partikel vorgegebener Größe, die eine Dichte von 1,0 g/cm³ bis 1,84 g/cm³ und eine Partikelgröße von 5-50 µm aufweisen, als Zusatz in einer kosmetischen Zusammensetzung, die das Aussehen eines Gels hat, die für Masken zur Reinigung der Gesichtshaut verwendet wird, um ihr die Eigenschaften einer Creme zu verleihen.

14. Verwendung nach Anspruch 13, dadurch gekennzeichnet, daß die Partikel in einem Anteil von mindestens 12 Gew.-%, bezogen auf das Gesamtgewicht der Zusammensetzung, enthalten sind.

15. Verwendung nach Anspruch 14, dadurch gekennzeichnet, daß die Partikel in einem Anteil von 12 bis 35 Gew.-%, bezogen auf das Gesamtgewicht der Zusammensetzung, enthalten sind.

16. Verwendung nach einem der Ansprüche 13 bis 15, dadurch gekennzeichnet, daß die Partikel eine Dichte von 1,02 bis 1,4 g/cm³ aufweisen.

17. Verwendung nach einem der Ansprüche 13 bis 16, dadurch gekennzeichnet, daß die Partikel eine Partikelgröße von 10 bis 30 µm aufweisen.

18. Kosmetische Verwendung der Zusammensetzung nach einem der Ansprüche 1 bis 12 zur Reinigung der Gesichtshaut.

19. Verfahren zur kosmetischen Behandlung, dadurch gekennzeichnet, daß auf die Gesichtshaut eine wie in einem der Ansprüche 1 bis 12 definierte kosmetische Zusammensetzung aufgetragen wird.
